Europäisches Patentamt

**European Patent Office**

Office européen des brevets

(11) Veröffentlichungsnummer: **0 031 294**
**A1**

(12) **EUROPÄISCHE PATENTANMELDUNG**

(21) Anmeldenummer: **80730076.9**

(22) Anmeldetag: **05.12.80**

(51) Int. Cl.³: **A 61 C 13/12**
**B 22 C 23/00, B 25 G 3/20**
**B 44 B 11/00**

(30) Priorität: **24.12.79 DE 2952103**
**24.12.79 DE 2952102**

(43) Veröffentlichungstag der Anmeldung:
**01.07.81 Patentblatt 81/26**

(84) Benannte Vertragsstaaten:
**AT BE CH DE FR GB IT LI LU NL SE**

(71) Anmelder: **ERSA Ernst Sachs KG GmbH & Co.**
**Leonhard-Karl-Strasse 24**
**D-6980 Wertheim / Main(DE)**

(72) Erfinder: **Hombrecher, Fritz**
**Oberer Neuberg 9**
**D-6980 Wertheim/Main(DE)**

(74) Vertreter: **Lüke, Dierck-Wilm, Dipl.-Ing.**
**Gelfertstrasse 56**
**D-1000 Berlin 33(DE)**

(54) Modelliergerät.

(57) Bei der Anfertigung von partiellen oder totalen Zahnprothesen werden Modelliergeräte verwendet, in deren Handgriff Modellierklingen oder dgl. eingesetzt sind. Diese sollen möglichst einfach und schnell auswechselbar sein, insbesondere dann, wenn diese auf Arbeitstemperaturen zwischen 60 und 140°C aufgeheizt sind. Das Modelliergerät weist hierzu eine Klemmeinrichtung (4) für den Schaft (5) der Modellierklingen (6) auf, welche einhändig durch Daumendruck auf das entgegengesetzte Ende des Handgriffes (2) betätigbar ist. Hierdurch können insbesondere auch auf Arbeitstemperatur befindliche Modellierklingen (6) und dgl. ohne Zuhilfenahme von Faßzangen ausgewechselt werden. Ferner ist ein sehr zweckmässiges Aufbewahrungsgerät für Modellierklingen und dgl. vorgesehen, in welchem diese bereits auf Arbeitstemperatur aufgeheizt vorrätig gehalten werden und ohne Anfassen von Hand mittels des Modelliergerätes schnell und einfach entnommen werden können. Beide Geräte bilden eine bauliche Einheit.

*FIG. 3*

0031294

## Patentanwälte
## Dipl.-Ing. Hans Albrecht
## Dipl.-Ing. Dierck-Wilm Lüke

Albrecht & Lüke, Gelfertstr. 56, D–1000 Berlin 33

Gelfertstraße 56
D–1000 Berlin 33
Telefon: (030) 8 31 30 28
Telegramme: Patentalbrecht Berlin

| Ihr Zeichen | Ihre Nachricht | Unser Zeichen | Datum |
|---|---|---|---|
| | | 10120 /L/Lü | 5. Dezember 1980 |

ERSA Ernst Sachs KG GmbH & Co., Leonhard - Karl Str. 24,

6980 Wertheim/ Main

-----------------------------------------------------------------

M O D E L L I E R G E R Ä T

-----------------------------------------------------------------

Die Erfindung bezieht sich auf ein Modelliergerät , insbesondere zur Wachsbearbeitung an Modellen für die Anfertigung von partiellen oder totalen Zahnprothesen, mit einem dünnen Trägerrohr für Modellierklingen und mit einem Handgriff.

Für die Bearbeitung der mit Wachs angefertigten Modelle werden die Modellierklingen auf Temperaturen von etwa 60 - 140 °C aufgeheizt. Dieses erfolgt entweder durch Erwärmung über offenen Flammen oder durch elektrische Beheizung der

Modelliergeräte, wozu am freien, mit der Modellierklinge versehenen Ende des Modelliergerätes eine Heizwendel in das Trägerrohr eingesetzt ist und am anderen Ende ein elektrisches Zuleitungskabel in das Trägerrohr geführt ist.

Bei der Arbeit mit den Modelliergeräten müssen die auf die genannten Temperaturen erhitzten Modellierklingen häufig ausgewechselt werden, um mit unterschiedlich geformten Modellierklingen am Modell arbeiten zu können. Dieser häufige Wechsel aufgeheizter Modellierklingen erfordert die Verwendung von Faßzangen, da die erhitzten Modellierklingen von Hand nicht angefasst werden können. Dabei ist das Aufsetzen und Abnehmen der Modellierklingen vom Modelliergerät äusserst umständlich und zeitraubend, da bei bekannten Modelliergeräten das Trägerrohr in eine Bohrung des Schaftes der Modellierklingen eingesteckt werden muß. Außerdem ergeben sich Wartezeiten durch den Aufheizzeitraum für die ausgewechselte Modellierklinge. Um einen häufigen Wechsel der Modellierklingen zu vermeiden, werden daher häufig mehrere Modelliergeräte mit verschieden geformten Modellierklingen verwendet. Dies bedeutet einen erhöhten Aufwand an Anschaffungs- und Energiekosten für mehrere Modelliergeräte.

Der Erfindung liegt die Aufgabe zugrunde, ein Modelliergerät der eingangs genannten Art zu schaffen, bei welchem das Auswechseln der Modellierklingen einfach und schnell möglich ist und das darüberhinaus preiswert in der Herstellung ist,sowie Wartezeiten für ein erneutes Aufheizen nach dem Wechseln einer Modellierklinge vermeidet.

Zur Lösung dieser Aufgabe sieht die Erfindung vor, dass das Trägerrohr innerhalb einer als Handgriff dienenden Hülse längs-verschiebbar gelagert ist und dass an einem Ende der Hülse eine durch Längsverschiebung des Trägerrohres innerhalb der Hülse zu betätigende Klemmeinrichtung für den Schaft der Modellierklingen angeordnet ist. Durch die Verwendung einer einfach zu betätigenden Klemmeinrichtung für die Modellierklingen wird das Auswechseln der Modellierklingen vereinfacht und kann äusserst schnell durchgeführt werden. Werden die einzelnen Modellierklingen beispielsweise in einem Aufbewahrungsgerät beheizt und so vorrätig gehalten, dass der Schaft jeder Modellierklinge nach oben ragt, so kann in einfacher Weise ein Wechsel der einzelnen Modellierklingen erfolgen, ohne dass diese überhaupt angefasst werden müssen.

Das erfindungsgemässe Arbeitsgerät kann bei elektrischer Beheizung des mit der Modellierklinge verbundenen freien Endes des Trägerrohres durch eine Heizwendel auch zur Aufnahme von Lötspitzen dienen und somit als Lötkolben mit Schnellwechseleinrichtung für die Lötspitzen verwendet werden. In entsprechender Weise können auch Brennadeln eingesetzt werden, so dass das erfindungsgemässe Arbeitsgerät dann als Brenneinrichtung für die Herstellung von Brennzeichnungen in Holz, Leder oder dgl. verwendet werden kann.

Da es wegen der Arbeitstemperaturen zwischen etwa 60 und 140 °C nahezu unmöglich ist , die Modellierklingen von Hand mit ihrem Schaft in das Modelliergerät einzuführen , ist zur Aufnahme der Modellierklingen und zu deren Entnahme mittels des Modelliergerätes ein Aufbewahrungsbehälter vorgesehen , dessen Merkmale sich aus den Unteransprüchen 9 bis 11 ergeben . Durch Beheizung des innerhalb der Wanne befindlichen Wachses auf die gewünschte Arbeitstemperatur können die in dem innerhalb der Wanne befindlichen Köcher mit nach oben aus der Wanne ragendem Schaft angeordneten Modellierwerkzeuge mittels des erfindungsgemäßen Modelliergerätes unmittelbar mit der gewünschten Arbeitstemperatur entnommen werden und müssen nicht erst zum Gebrauch auf die Arbeitstemperatur aufgeheizt werden . Das Entnehmen erfolgt dabei durch Einhandbedienung des Modelliergerätes , ohne daß die Modellierwerkzeuge von Hand angefaßt werden müssen .Da die Arbeitswerkzeuge bereits auf die Arbeitstemperatur aufgehe kann eine besondere Beheizung durch offene Flammen oder durch die in die Modelliergeräte eingebaute elektrische Heizung entfallen .

Die Erfindung ist nachfolgend anhand eines in den Zeichnungen dargestellten Ausführungsbeispieles näher erläutert. Es zeigen:

Fig. 1  eine Seitenansicht eines Modelliergerätes etwa in natürlicher Grösse,

Fig. 2 und 3  Längsschnitte durch das vordere Ende des Modelliergerätes (etwa M 2:1 ),

Fig. 4    einen Längsschnitt durch das hintere Ende des Modelliergerätes in etwa natürlicher Grösse,

Fig. 5    eine perspektivische Ansicht auf ein Aufbewahrungsgerät mit daran befindlichem Modelliergerät und

Fig. 6    einen Längsschnitt durch das Aufbewahrungsgerät gemäss Fig. 5 mit elektronischer Regeleinrichtung.

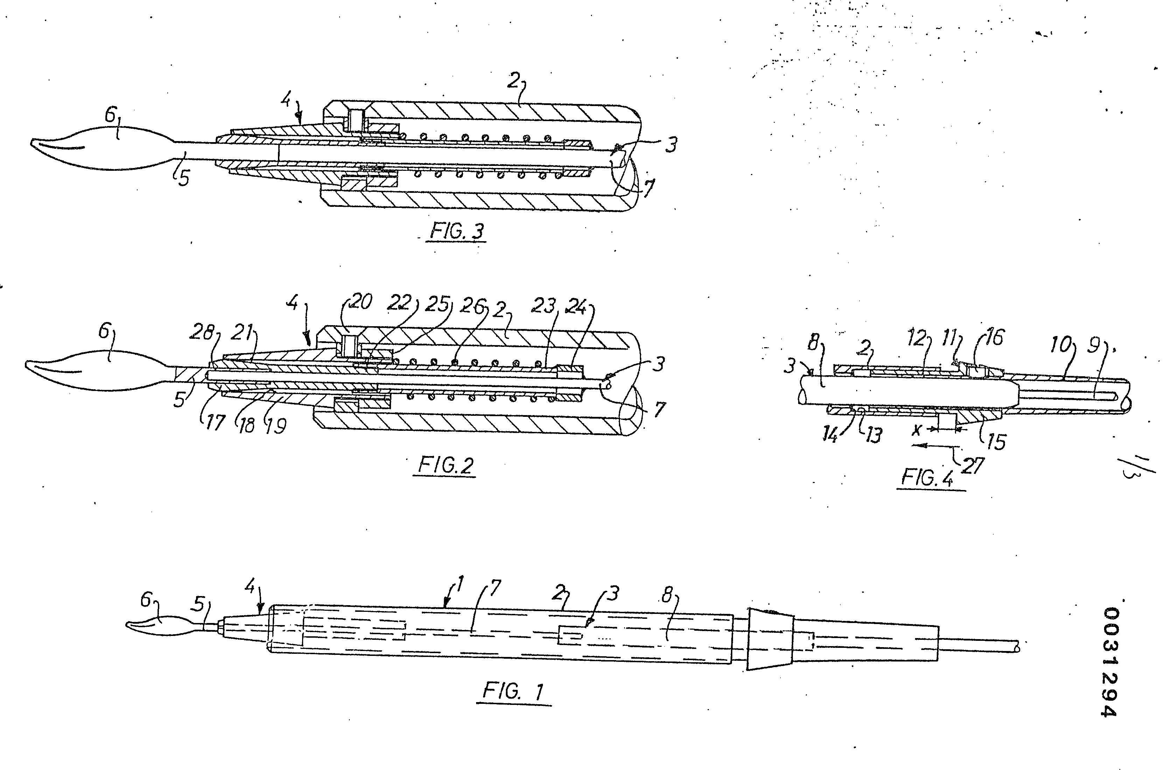

Das in Fig. 1 dargestellte Modelliergerät 1 umfasst eine als Handgriff dienende Hülse 2, ein innerhalb der Hülse 2 längs - verschiebbar gelagertes Trägerrohr 3 und eine durch Längsverschiebung des Trägerrohres 3 innerhalb der Hülse 2 zu

betätigende Klemmeinrichtung 4 für den Schaft 5 von Modellierklingen 6. Die Hülse 2 ist als langgestrecktes, dünnwandiges Rohr, insbesondere aus Kunststoff ausgebildet.

Das Trägerrohr 3 entspricht der Ausbildung eines innenbeheizten elektrischen Kleinstlötkolbens für Kleinspannung gemäss DE-PS 1.248.829. Dabei umfasst das Trägerrohr 3 einen Rohrabschnitt 7 mit einem Aussendurchmesser von etwa 1,5 mm und einem Innendurchmesser von etwa 1 mm, in dessen freies, zur Modellierklinge 6 hin gerichtetes Ende eine Heizwendel mit Verbindungsleitungen eingesetzt ist, und einen wesentlich dickeren, insbesondere aus Kunststoff ausgebildeten Rohrabschnitt 8 zur Aufnahme des dünnen Rohrabschnittes 7 und zur Aufnahme eines elektrischen Zuleitungskabels 9, sowie zur Verbindung der Enden des Zuleitungskabels 9 mit den Zuleitungsdrähten zur Heizwendel. Das hintere Ende des dicken Rohrabschnittes 8 ragt aus dem hinteren Ende der als Handgriff dienenden Hülse 2 heraus und ist von einer das elektrische Zuleitungskabel 9 schützenden Knickschutztülle 10 sowie einem Betätigungselement 11 für die Längsverschiebung des Trägerrohres 3 innerhalb der Hülse 2 umgeben. Das Betätigungselement 11 umfasst eine Schiebehülse 12, die innerhalb einer Ausdrehung 13 der Hülse 2 bis zu einem Anschlag 14 längsverschieblich innerhalb der Hülse 2 gelagert ist. Ausserhalb der Hülse 2 umfasst das Betätigungselement 11 einen umlaufenden Bund 15 zur Betätigung mittels Daumen oder Daumen und Zeigefinger einer Bedienungsperson. Mittels einer Madenschraube 16 sind die Knickschutztülle 10 und das Betätigungselement 11 am dickeren Rohrabschnitt 8 des Trägerrohres 3 festgelegt. Der umlaufende Bund 15 des Betätigungselementes 11 ist um das Maß x ( Fig. 4) in Längsrichtung der Hülse 2 gegenüber dieser bewegbar, womit gleichzeitig das Trägerrohr 3 um das gleiche Maß x gegenüber der Hülse 2 bewegbar ist.

Die Klemmeinrichtung 4 besteht aus einer den Schaft 5 der Modellierklingen 6 und den dünnen Rohrabschnitt 7 des Träger-rohres 3 umgreifenden beweglichen Spannzange 17 und aus einer an der Hülse 2 festgelegten, die Spannzange 17 in einer Innenbohrung 18 aufnehmenden Zangenaufnahme 19, die durch einen Stift oder Schraube 20 innerhalb der Innenbohrung der Hülse 2 festgelegt ist. Die Spannzange 17 besteht aus mehreren,vzgws. vier federnden Zangenelementen, welche den Schaft 5 der Modellierklingen 6 symmetrisch umgeben. Die einzelnen Elemente der Spannzange 17 umfassen Schrägflächen 21, welche beim Einfahren der Spannzange 17 in die Innenbohrung 18 der Zangenaufnahme 19 am inneren Umfang der Innenbohrung 18 anliegend radial zusammengedrückt werden , und so den Schaft 5 der Modellierklingen 6 festumfassen. Die Zangenaufnahme 19 ist aussen in Richtung auf die Modellierklingen 6 hin konisch ausgebildet.

Die einzelnen Zangenelemente der Spannzange 17 sind am unteren Ende in einem ringförmigen Fußteil 22 zusammengefasst, von welchem die Zangenelemente zungenförmig ausgehen. Auf das ringförmige Fußteil 22 ist ein Verlängerungsrohr 23 aufgeschraubt, das mit einem an der Spannzange 17 entgegengesetzt liegenden Ende angeordneten, am dünnen Rohrabschnitt 7 des Trägerrohres 3 mittels Hartlötens oder Verschweissens festgelegten Gegenlager 24 versehen ist. Zwischen dem Gegenlager 24 und dem in die Hülse 2 gerichteten Bund 25 der Zangenaufnahme 19 ist eine Schraubenfeder 26 angeordnet, unter deren Wirkung die Spannzange 17 mit ihren einzelnen Zangenelementen in die Innenbohrung 18 der Zangenaufnahme 19 hineingezogen wird.

Gleichzeitig wird unter Wirkung der Feder 26 das Trägerrohr 3 in Richtung auf das durch das Betätigungselement 11 gekennzeichnete Ende verschoben, so dass der Bund 15 des Betätigungselementes 11 im Abstand des Maßes x vom freien Ende der Hülse 2 angeordnet ist.

Ein Druck in Richtung des Pfeiles 27 auf den Bund 15 des Betätigungselementes 11 bewirkt eine Verschiebung des gesamten Trägerrohres 3 in Richtung auf die Klemmeinrichtung 4, wobei deren Klemmzange 17 teilweise aus der Zangenaufnahme 19 herausgeschoben wird, so dass der Schaft 5 der Modellierklingen 6 von den Spannzangenelementen 17 freikommt und zum Austausch der Modellierklingen 6 herausgenommen werden kann.

In der Ausführungsform gemäss Fig. 2 taucht das freie Ende des Innenrohrabschnittes 7 des Trägerrohres 3, in welchen die Heizwendel eingesetzt ist, in eine Innenbohrung 28 innerhalb des Schaftes 5 der Modellierklingen 6 ein, um diese unmittelbar zu beheizen. In der Ausführungsform gemäss Fig. 3 stösst das freie Ende 6 des Trägerrohres 3 an das freie Ende des Schaftes 5 an, wobei die Wärme der in das Trägerrohr 3 eingesetzten Heizwendel über die einzelnen Spannzangenelemente auf den Schaft 5 und somit auf die Modellierklingen 6 übertragen wird.

Das hier beschriebene Modelliergerät kann mit seiner Klemmeinrichtung 4 und dem in die Hülse 2 längsverschiebbar eingesetzten Trägerrohr 3 sowie dem Betätigungselement 11 auch ohne elektrische Zuleitungskabel 9 und dieses umgebende Knickschutztülle 10 sowie ohne in das Trägerrohr 3 eingebaute Heizwendel mit gleichen Vorteilen verwendet werden.

Bei Verwendung des Modelliergerätes 1 im Dentallabor zur Wachsbearbeitung an Modellen bei der Anfertigung von partiellen oder totalen Zahnprothesen können die einzelnen Bauteile der Klemmeinrichtung 4, d.h. insbesondere die Klemmzangen 17 und die Zangenaufnahme 19 aus Hartmessing bestehen, da nur Temperaturbereiche zwischen etwa 60 und 140°C auftreten können. Diese Temperaturen reichen zur Benutzung der Modellierklingen oder auch Wachsspachtel genannt aus.

Zur Verwendung beim Löten und Brennen müssen wärmebeständigere Materialien für die einzelnen Bauteile , insbesondere die Spannzange 17 und die Zangenaufnahme 19 gewählt werden, wie insbesondere gehärteter Stahl.

In allen Anwendungsfällen ist ein einfaches Auswechseln der Modellierklingen bzw. Wachsspachtel, der Lötspitzen und Brennspitzen gewährleistet.

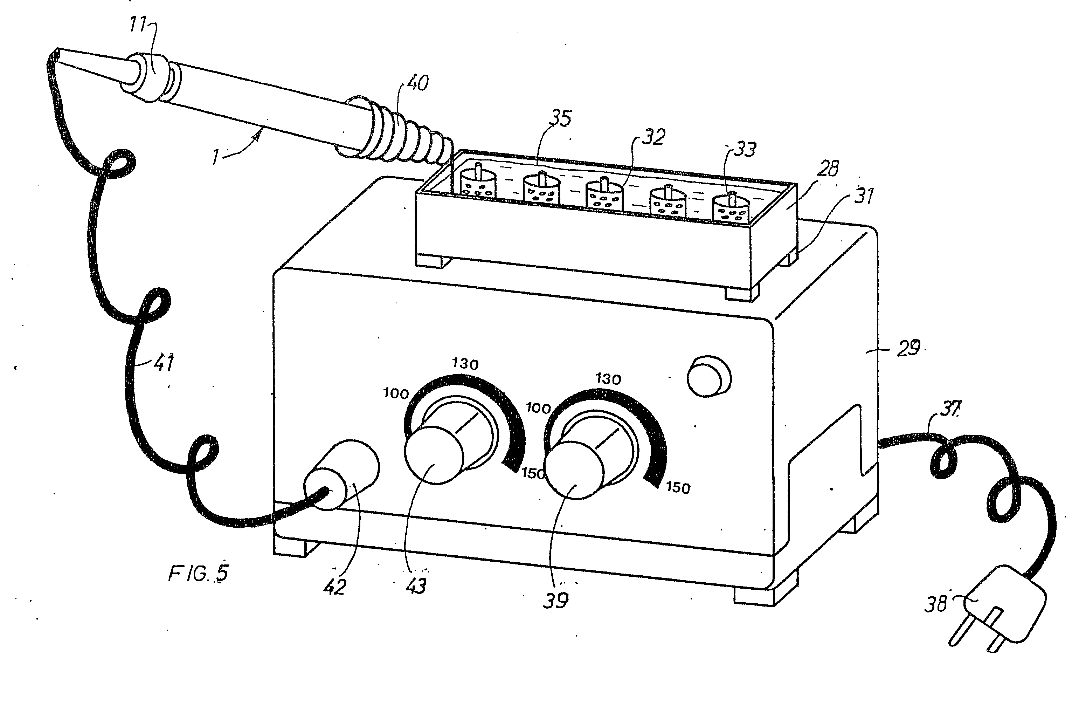

Das erfindungsgemässe Modelliergerät 1 kann insbsondere zusammen mit dem in Fig. 5 perspektivisch dargestellten Aufbewahrungsgerät für Modellierklingen, Sonden und dgl.

und für das Modelliergerät 1 selbst verwendet werden. Das Aufbewahrungsgerät umfasst eine mit einem flüssigen Medium angefüllte Wanne 28 und ein Gehäuse 29 für eine elektronische Regeleinrichtung 30. Die Wanne 28 ist unter Zwischenlage von Wärmeisolatoren 31 auf der oberen Wand des Gehäuses 29 angeordnet.

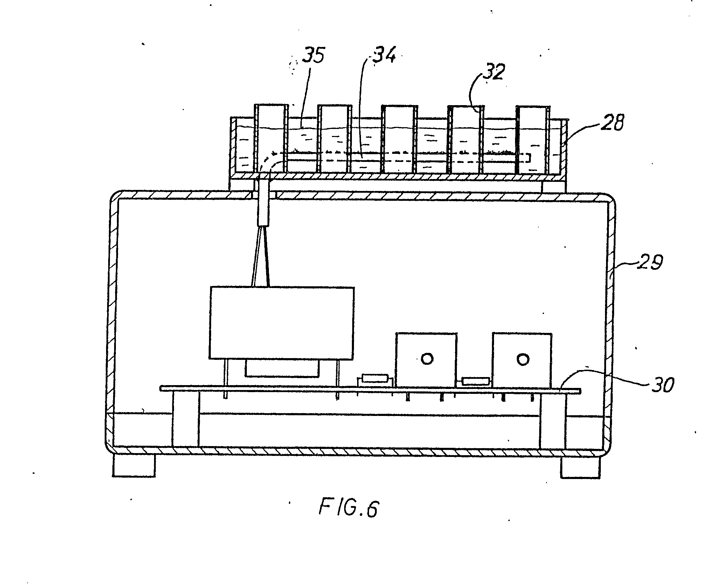

In der als flüssigkeitsdichter, rechteckiger und nach oben vollgeöffneten Wanne 28 aus Metall sind in Abständen von einander Köcher 32 zur Aufnahme der Modellierklingen, Sonden oder dgl. 33 angeordnet, die mit ihrem Schaft nach oben aus den Köchern 32 und damit aus der Wanne 28 herausragen. Die Köcher 32 sind aus senkrecht auf dem Boden der Wanne 28 stehenden, mit Öffnungen versehenen Blechzylindern gebildet. Parallel zum Boden der Wanne 28 erstreckt sich als Heizeinrichtung für das in der Wanne 28 befindliche flüssige Wachs ein Heizstab 34. Dieser kann das Wachs auf die Arbeitstemperatur für die Modellierklingen, Sonden od. dgl., d.h. auf eine Temperatur zwischen etwa 60 und 140°C erwärmen, in welchem Fall das Wachs dann die Wanne 28 bis zum Wachsspiegel/anfüllt. Die Wanne 28 ist unter Zwischenlage der 35 Wärmeisolatoren 31 unmittelbar auf die obere Wand des Gehäuses 29 für die elektronische Regeleinrichtung 30 aufgesetzt ( Fig.6). Der Heizstab 34 ist senkrecht zum Boden der Wanne 28 abgebogen, durchdringt diesen flüssigkeitsdicht und ragt nach unten in das Gehäuse 29 hinein.

Innerhalb des Gehäuses 29 ist die für die Beheizung des flüssigen Mediums innerhalb der Wanne 28 dienende elektronische Regeleinrichtung 30 auf einer Platine in bekannter Bauart angeordnet und mit dem Heizstab 34 sowie in einem elektrischen Zuleitungskabel 37 mit Stecker 38 verbunden. In der Stirnseite des Gehäuses 29 ist ein Regelknopf 39 zur Temperatureinstellung des flüssigen Wachses innerhalb der Wanne 28 angeordnet. Der Regelknopf 39 wirkt unmittelbar auf ein Bauteil der elektronischen Regeleinrichtung 30 für den Heizstab 34 ein.

Am Gehäuse 29 ist eine Aufnahme 40 für das Modelliergerät 1 vorgesehen, das über ein Zuleitungskabel 41 mit einem Stecker 42 verbunden ist, der eine elektrische Verbindung mit der elektronischen Regeleinrichtung 30 innerhalb des Gehäuses 29 ermöglicht. Diese weist zusätzliche Bauteile für die Regelung der Temperatur der Modellierklinge 6 des Modelliergerätes 1 auf. Für die Temperaturregelung des Modelliergerätes 1 ist zusätzlich ein Regelknopf 43 in der Stirnseite des Gehäuses 29 angeordnet, der in unmittelbarer elektrischer Verbindung mit den zugehörigen Bauteilen der elektronischen Regeleinrichtung 30 innerhalb des Gehäuses 29 steht.

Die Wanne 28 kann gleichzeitig als elektrisches Wachswarmhaltegerät dienen und hierzu erforderlichenfalls
auch in mehrere Wannenbecken für verschiedene Wachssorten
unterteilt sein. In weiterhin nicht näher dargestellter
Weise kann die Wanne 28 auch als gegenüber dem Gehäuse
29 für die elektronische Regeleinrichtung 30 getrenntes
Bauteil ausgebildet sein, wobei lediglich eine elektrische
Verbindung zum Heizstab 34 innerhalb der Wanne 28 vorgesehen
sein muss.

Patentanwälte
Dipl.-Ing. Hans Albrecht
Dipl.-Ing. Dierck-Wilm Lüke

Albrecht & Lüke, Gelfertstr. 56, D–1000 Berlin 33

Gelfertstraße 56
D–1000 Berlin 33
Telefon: (030) 8 31 30 28
Telegramme: Patentalbrecht Berlin
Postscheck: Berlin West 336 26-105
Bank: Berliner Bank AG
Konto-Nr. 43 09 53 99 00

| Ihr Zeichen | Ihre Nachricht | Unser Zeichen | Datum |
|---|---|---|---|
| | | 10120/L/LÜ | 5. Dezember 1980 |

Patentansprüche

-------------------------------

1. Modelliergerät, insbesondere zur Wachsbearbeitung an Modellen für die Anfertigung von partiellen oder totalen
Zahnprothesen, mit einem dünnen Trägerrohr für Modellierklingen und einem Handgriff, dadurch gekennzeichnet, dass
das Trägerrohr (3) innerhalb einer als Handgriff dienenden
Hülse (2) längsverschiebbar gelagert ist und dass an einem
Ende der Hülse (2) eine durch Längsverschiebung des Trägerrohres (3) innerhalb der Hülse (2) zu betätigende Klemmeineinrichtung (4) für den Schaft (5) der Modellierklingen (6)
angeordnet ist.

2. Modelliergerät nach Anspruch 1, dadurch gekennzeichnet,
dass die Klemmeinrichtung (4) aus einer den Schaft (5) der

Modellierklingen (6) und das Trägerrohr (3) umgreifenden, beweglichen federnden . Spannzange (17) und aus einer an der Hülse (2) festgelegten, die Spannzange (17) in einer Innenbohrung (18) aufnehmenden Zangenaufnahme (19) gebildet ist und dass die Spannzange (17) zur Klemmung des Schaftes (5) der Modellierklingen (6) unter Wirkung einer Feder (26) in die Zangenaufnahme (19) hineinziehbar und zum Lösen der Klemmung durch Betätigung des Trägerrohres (3) entgegen der Kraft der Feder (26) aus der Zangenaufnahme (19) herausbewegbar ist.

3. Modelliergerät · nach Anspruch 2, dadurch gekennzeichnet, dass die Spannzange (17) mit einem in die Hülse (2) hineinragenden, das Trägerrohr (3) teilweise in Längsrichtung umgebenden Verlängerungsrohr (23) mit einer am der Spannzange (17) entfernt liegenden Ende angeordneten, am Trägerrohr (3) festgelegten Gegenlager (24) versehen ist und dass die Feder (26) zwischen dem Gegenlager (24) und dem in die Hülse (2) gerichteten Bund (25) der Zangenaufnahme (19) angeordnet ist.

4. Modelliergerät nach Anspruch 2 oder 3, dadurch gekennzeichnet, dass das Trägerrohr (3) an dem der Klemmeinrichtung (4) entgegengesetzten Ende der Hülse (2) mit einem auf der Hülse (2) herausragenden Betätigungselement (11) versehen ist.

5. Modelliergerät nach einem der vorangegangenen Ansprüche, mit einer Heizwicklung am in der Klemmeinrichtung befindlichen freien Ende des Trägerrohres und mit einer elektrischen Zuleitung am entgegengesetzten Ende des Trägerrohres, dadurch gekennzeichnet, dass das Trägerrohr (3) in den Schaft (5) der Modellierklingen (6) eintauchbar ist .

6. Modelliergerät nach einem der Ansprüche 1 bis 4 , mit einer Heizwicklung am in der Klemmeinrichtung befindlichen freien Ende des Trägerrohres und mit einer elektrischen Zuleitung am entgegengesetzten Ende des Trägerrohres, dadurch gekennzeichnet, dass das Trägerrohr (3) und der Schaft (5) der Modellierklingen (6) innerhalb der Klemmeinrichtung (4) bündig aneinanderstossend angeordnet sind.

7. Modelliergerät nach einem der Ansprüche 1 bis 6 , dadurch gekennzeichnet, dass das Betätigungselement (11) als das freie, aus der Hülse (2) herausragende Ende des Trägerrohres (3) umgebender Bund (15) ausgebildet ist.

8. Modelliergerät nach Anspruch 7, dadurch gekennzeichnet, dass das Betätigungselement (11) mit einer rohrartigen Schiebehülse (12) in eine Ausdrehung der Hülse (2) hineinragt und um ein Maß (x) innerhalb der Hülse (2) bis zu einem Anschlag (14) entgegen der Wirkung der Feder (16) bewegbar ist.

9. Modelliergerät nach einem der vorangegangenen Ansprüche, dadurch gekennzeichnet, dass eine mit flüssigem Wachs angefüllte, mittels einer Heizeinrichtung (34) elektrische beheizbare Wanne (28) vorgesehen und in der Wanne (28) mindestens ein Köcher (32) zur Aufnahme von Modelliermessern oder dgl. (33) mit nach oben aus der Wanne (28) ragenden Schaft angeordnet ist.

10. Modelliergerät nach Anspruch 9, dadurch gekennzeichnet, dass die Wanne (28) unter Zwischenlage von Wärmeisolatoren (31) auf dem Gerätegehäuse (29) angeordnet und mit mehreren Köchern (32) versehen ist und dass die Heizeinrichtung als parallel zum Boden der Wanne (28) angeordnete Heizstab (34) ausgebildet ist.

11. Modelliergerät nach Anspruch 9 oder 10, dadurch gekennzeichnet, dass der bzw. die Köcher (32) aus senkrecht auf dem Boden der Wanne (28) stehenden, mit Wandöffnungen versehenen Blechzylindern ausgebildet sind.

FIG. 3

FIG.2

FIG. 4

FIG. 1

0031294

1/3

FIG.5

FIG.6

# EUROPÄISCHER RECHERCHENBERICHT

Europäisches
Patentamt

| | EINSCHLÄGIGE DOKUMENTE | | KLASSIFIKATION DER ANMELDUNG (Int. Cl.³) |
|---|---|---|---|
| Kategorie | Kennzeichnung des Dokuments mit Angabe, soweit erforderlich, der maßgeblichen Teile | betrifft Anspruch | |
| | DE - U - 1 740 885 (FISCHER et al.)<br>* ganzes Dokument *<br>--- | 1,5,<br>9,10 | A 61 C  13/12<br>B 22 C  23/00<br>B 25 G   3/20<br>B 44 B  11/00 |
| | CH - A5 - 563 847 (AEBI)<br>* Fig. *<br>-- | 1-3 | |
| A | DE - B - 1 248 829 (ERSA)<br>--- | | |
| A | DE -C - 1 116 118 (THUM)<br>-- | | RECHERCHIERTE SACHGEBIETE (Int. Cl.³) |
| A | DE - C - 565 506 (WARNIMONT)<br>-- | | A 61 C   1/00<br>A 61 C  13/00 |
| A | DE - C - 537 054 (WESTDEUTSCHER IN-<br>DUSTRIE OFENBAU)<br>-- | | A 61 C  19/00<br>B 22 C   7/00<br>B 22 C  23/00 |
| A | DE - C - 457 131 (DEUTSCHE GOLD- UND<br>SILBER-SCHEIDEANSTALT)<br>-- | | B 23 K   3/00<br>B 25 G   3/00<br>B 44 B  11/00 |
| A | DE - U1 - 7 830 972 (DEUTSCHE GOLD- UND<br>SILBER-SCHEIDEANSTALT)<br>-- | | KATEGORIE DER GENANNTEN DOKUMENTE |
| A | AT - B - 146 632 (VOKALEK)<br>-- | | X: von besonderer Bedeutung<br>A: technologischer Hintergrund<br>O: nichtschriftliche Offenbarung |
| A | DD - U - 12 523 (DIECKMANN)<br>---- | | P: Zwischenliteratur<br>T: der Erfindung zugrunde<br>liegende Theorien oder<br>Grundsätze<br>E: kollidierende Anmeldung<br>D: in der Anmeldung angeführtes<br>Dokument<br>L: aus andern Gründen<br>angeführtes Dokument<br>&: Mitglied der gleichen Patent- |

Der vorliegende Recherchenbericht wurde für alle Patentansprüche erstellt.

familie, übereinstimmendes
Dokument

| Recherchenort | Abschlußdatum der Recherche | Prüfer |
|---|---|---|
| Berlin | 16-03-1981 | SIMON |

EPA form 1503.1  06.78